(19) **Europäisches Patentamt European Patent Office Office européen des brevets**

(11) **EP 3 552 609 B1**

(12) **EUROPEAN PATENT SPECIFICATION**

(45) Date of publication and mention of the grant of the patent:
**03.05.2023 Bulletin 2023/18**

(21) Application number: **17878030.0**

(22) Date of filing: **08.12.2017**

(51) International Patent Classification (IPC):
*A61K 33/30* (2006.01)   *A61K 45/06* (2006.01)
*A61L 15/18* (2006.01)   *A61P 17/02* (2006.01)

(52) Cooperative Patent Classification (CPC):
**A61K 33/30; A61K 45/06; A61L 15/18; A61P 17/02**

(86) International application number:
**PCT/JP2017/044220**

(87) International publication number:
**WO 2018/105738 (14.06.2018 Gazette 2018/24)**

(54) **ZINC CHLORIDE HYDROXIDE HAVING EXCELLENT ZINC ION SUSTAINED-RELEASE AND ITS METHOD OF MANUFACTURE**

ZINKCHLORIDHYDROXID MIT HERVORRAGENDER VERZÖGERTER FREISETZUNG VON ZINKIONEN SOWIE DESSEN HERSTELLUNG

HYDROXYDE DE CHLORURE DE ZINC AYANT UNE EXCELLENTE LIBÉRATION PROLONGÉE D'IONS ZINC ET SON PROCÉDÉ DE PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2016 JP 2016239802**

(43) Date of publication of application:
**16.10.2019 Bulletin 2019/42**

(73) Proprietor: **JFE Mineral Company, Ltd.**
**Tokyo 105-0014 (JP)**

(72) Inventors:
• **NAKATA, Yoshimi**
**Tokyo 105-0014 (JP)**
• **ECHIZENYA, Yuko**
**Tokyo 105-0014 (JP)**
• **UDAGAWA, Etsurou**
**Tokyo 105-0014 (JP)**
• **YAMAMOTO, Osamu**
**Yonezawa City**
**Yamagata 992-8510 (JP)**

(74) Representative: **Grünecker Patent- und**
**Rechtsanwälte**
**PartG mbB**
**Leopoldstraße 4**
**80802 München (DE)**

(56) References cited:
WO-A1-2012/068620    WO-A1-2016/199905
CN-A- 1 569 643    US-A1- 2016 326 006

• IOANNIS MAVROMICHALIS ET AL: "Growth-promoting efficacy of pharmacological doses of tetrabasic zinc chloride in diets for nursery pigs", CANADIAN VETERINARY JOURNAL - REVUE VETERINAIRE CANADIENNE, vol. 81, no. 3, 1 September 2001 (2001-09-01), pages 387-391, XP055333642, CA ISSN: 0008-5286, DOI: 10.4141/A01-005
• EUROPEAN FOOD SAFETY AUTHORITY (EFSA): 'Scientific opinion on the safety and efficacy of tetra-basic zinc chloride for all animal species' EFSA JOURNAL vol. 10, no. 5, 01 May 2012, page 2672, XP055511627
• COUSY, SIMON ET AL.: 'Basic precipitation of simonkolleite nanoplatelets' 2013 NANOCON, [Online] 18 October 2013, CZECH REPUBLIC, XP055511634 Retrieved from the Internet: <URL:http://konsys-t.tanger.cz/files/procee dings/14/reports/2061.pdf> [retrieved on 2018-01-16]

EP 3 552 609 B1

## Description

TECHNICAL FIELD

The present invention relates to a kit-of-parts comprising a zinc chloride hydroxide having excellent zinc ion sustained-release properties and a wound covering material and a method for manufacturing the zinc chloride hydroxide.

BACKGROUND ART

Patent Literature 1 describes in paragraph [0013] that, regarding the zinc supplements, "it is found that administration of relatively high amounts of well absorbed forms of zinc prior to, or concurrently with therapeutic administration of botulinus toxin will enable responsiveness to the toxin in individuals who were previously poorly responsive, and apparently enhance the functional potency of botulinus toxins in other individuals as well". Paragraph [0045] of Patent Literature 1 describes, as examples of such "zinc supplements", inorganic zinc or organic zinc or a combination thereof for oral administration, and zinc chloride ($ZnCl_2$), basic zinc chloride ($Zn_5Cl_2(OH)_8$), zinc oxide (ZnO), and zinc sulfate ($ZnSO_4$) are listed.

However, use of basic zinc chloride such as simonkolleite as an active ingredient of pharmaceuticals has not been studied. Basic zinc chloride is, for example, described by Mavromichalis et al. (Mavromichalis I. et al., Can. J. Anim. Sci., 2001, 81:387-391) as having a growth promoting effect at pharmacological doses in diets for nursery pigs. The preparation of simonkolleite nanoplatelets is described by Cousy et al. (Cousy S. et al., Nanocon, 2013).

Zinc is believed to be a substance that supplies $Zn^{2+}$ ions to a wound site when used as a therapeutic agent for a skin wound or skin roughness. A technique in which $Zn^{2+}$ ions can be appropriately supplied to a wound site, from the perspective of an active ingredient of pharmaceuticals, the substance can be supplied in the form of zinc chloride hydroxide having excellent stability, and in addition, the substance can be provided through a simple and convenient manufacturing method, is desired.

CITATION LIST

PATENT LITERATURE

Patent Literature 1: JP 2012-531421 A

SUMMARY OF INVENTION

TECHNICAL PROBLEM

The invention aims to provide a kit-of-parts comprising a zinc chloride hydroxide useful as an active ingredient of pharmaceuticals, having excellent zinc ion sustained-release properties and having excellent stability as an active ingredient of pharmaceuticals, when used as pharmaceuticals, and a method for manufacturing the same.

SOLUTION TO PROBLEMS

The active ingredient of pharmaceuticals mentioned later is characterized as an inorganic compound composition containing a zinc chloride hydroxide that can be used in the pharmaceuticals described below. Generally, inorganic compound compositions which contain zinc are of a plate-like shape and they are known to be used in cosmetics (applications to the skin) utilizing their concealing properties. However, to be used as an active ingredient of pharmaceuticals, a plate-like shape may cause a risk of cytotoxicity, and thus, it is preferably an atypical shape, an ellipsoid shape, a substantially spherical shape without sharp parts with a particle size of from 50 to 0.1 um, and more preferably a particle size of from 10 to 1 $\mu$m. More preferably, depending on the application of the pharmaceutical product, adjustments such as particle size, surface area, particle size distribution, may be made so that the sustained-release of zinc ions from the pharmaceuticals can be appropriately controlled.

The present invention relates to a kit-of-parts comprising a zinc chloride hydroxide and a chitin wound covering material as specified in the claims.

The invention further relates to a method for manufacturing a zinc chloride hydroxide as specified in the claims.

For the ultimate use as an active ingredient of pharmaceuticals, the manufacturing method described below comprises: a step of crushing for adjusting into preferably an atypical shape, an ellipsoid shape, or a substantially spherical shape without sharp parts with a particle size of from 50 to 0.1 um, and more preferably a particle size of from 10 to 1 um, or a step of adjusting the particle size using a ball mill or the like (including granulation). The method of

2

manufacturing the zinc chloride hydroxide according to the invention comprises maintaining an almost constant pH by acid or alkali in the presence of aqueous solution including chloride ions, continuously supplying zinc ions from an aqueous zinc solution, and precipitating zinc chloride hydroxide having a main component of simonkolleite. In the present description, "maintaining an almost constant pH" is not particularly limited and means maintaining preferably at $\pm 1$ of a predetermined pH and more preferably at $\pm 0.3$ of a predetermined pH. The "main component of simonkolleite" means that zinc chloride hydroxide contains simonkolleite as the most dominant component. Zinc chloride hydroxide may also be referred to as basic zinc hydroxide in the present description.

According to the invention, the pH is not less than 6.0 and less than 7.5, and a temperature is lower than 40°C.

ADVANTAGEOUS EFFECTS OF INVENTION

The invention is a kit-of-parts comprising a zinc chloride hydroxide which is useful as an active ingredient of pharmaceuticals and has an excellent stability and a chitin wound covering material.

BRIEF DESCRIPTION OF DRAWINGS

[FIG. 1] FIG. 1 is a chart of XRD (X-ray diffraction method) of simonkolleite obtained by the method for manufacturing a Preparation Example of the invention. pH is the pH when synthesized.

[FIG. 2] FIG. 2 shows photographs showing the observation results of a healing site (sample) of a wound site and of a control after one week in Pharmaceutical Example 1.

[FIG. 3] FIG. 3 shows photographs showing the observation results of the healing site (sample) and of the control after two weeks in Pharmaceutical Example 1.

[FIG. 4] FIG. 4 is a graph showing the results of Table 2.

[FIG. 5] FIG. 5 shows micrographs showing histological assessment of a wound created part after one week of treatment using Pharmaceutical Example 1.

[FIG. 6] FIG. 6 shows micrographs showing histological assessments of a wound created part 2 and a wound created part 3 after two weeks of treatment in Pharmaceutical Example 1. Part 1, part 4, and part 5 other than the wound created parts are shown together.

[FIG. 7] FIG. 7 is a graph showing the relationship between pH of simonkolleite at synthesis and pH after a dissolution test.

[FIG. 8] FIG. 8 is a graph showing the relationship between pH of simonkolleite at synthesis and the amount of dissolved $Zn^{2+}$ ions after the dissolution test.

DESCRIPTION OF EMBODIMENTS

1. Summary of Zinc chloride hydroxide of the Invention

(1) The zinc chloride hydroxide of the invention is represented by the chemical formula $Zn_{4-6}Cl_{1-3}(OH)_{7-8} \cdot nH_2O$ ... Formula (1), where n is 0 to 6.

In the industrial field, it is known as a generally representative chemical formula: simonkolleite $Zn_5(OH)_8Cl_2$, and this substance is known to be a dense corrosion product, have an excellent corrosion inhibitive effect, and improve corrosion resistance of a plating layer by promoting the generation of simonkolleite.

The zinc chloride hydroxide of the invention may be obtained using a precipitate produced by an alkali precipitation method from an aqueous zinc salt solution. Preferably, in the precipitate generation reaction described below, a precipitate, obtained by the reaction of $Zn^{2+}$ ions, $Cl^-$ ions, and $OH^-$ ions in a reaction field where the pH is controlled preferably not less than 6.0 and less than 7.5, is used as the zinc chloride hydroxide of the invention.

The zinc chloride hydroxide of the invention has an amount of dissolved $Zn^{2+}$ ions of from 0.25 to 100 $\mu g/m^2$, and pH of not less than 7.0 and less than 8.3, and more preferably an amount of dissolved $Zn^{2+}$ ions of from 10 to 100 $\mu g/m^2$ and pH of not less than 7.0 and less than 8.3 after a dissolution test by the stirring method explained in the Pharmaceutical Examples.

(Dissolution Test by Stirring Method)

The amount of dissolved $Zn^{2+}$ ions is measured in the present description as follows: The surface areas of samples, which are produced with varying pH values at production by the same process as that for Preparation Example 2 of Examples to be described later, are measured beforehand by the BET method (BET specific surface area analyzer: High Precision, Multi-Analyte Gas Adsorption Analyzer, available from Quantachrome Instruments Japan G.K.). For each sample, the $Zn^{2+}$ ion concentration after stirring in saline is measured by an ICP emission spectrometer (ICPE-9000, available from Shimadzu Corporation) to thereby obtain the amount of dissolved $Zn^{2+}$ ions, and the amount of dissolved $Zn^{2+}$ ions is divided by the surface area previously measured. The mass ratio of each sample to saline is 1:50 and the amount of $Zn^{2+}$ ions dissolved in the saline is measured after stirring at 37°C for 3 hours at 500 rpm using a rotor.

(2)
(2-1) The zinc chloride hydroxide is preferably coated, impregnated, or adhered to the wound covering material that retains the therapeutic agent for a skin wound or skin roughness in a closed environment.

(2-2) In another aspect, the pharmaceuticals may be used as a medical set for treating a skin wound or skin roughness, which is a combination of a therapeutic agent for a skin wound or skin roughness containing an zinc chloride hydroxide that can be used as an active ingredient of pharmaceuticals, with the above-mentioned wound covering material.

2. Method for Manufacturing zinc chloride hydroxide of the Invention

The chlorine source used in the alkali precipitation method is preferably an aqueous solution of at least one of NaCl or $NH_4Cl$ (ammonium chloride), and more preferably an aqueous $NH_4Cl$ solution. NaOH is added dropwise as a mineralization material so that the pH is maintained preferably at not less than pH 6.0 and less than 7.5, and an acidic aqueous solution of zinc salt such as zinc chloride is added dropwise to form a precipitate, and stirred for 10 to 30 hours to obtain a precipitate. After solid-liquid separation by suction filtration or centrifugation, the precipitate is washed with pure water or distilled water, and dried under vacuum to obtain a dry powder containing simonkolleite. The particle size of the obtained zinc chloride hydroxide hydrate containing simonkolleite is not limited, and when used as an active ingredient of pharmaceuticals such as therapeutic drugs, the particle size can be set to an appropriate particle size by a known method. The chlorine source includes aqueous solution of NaCl or the like, preferably $NH_4Cl$; the zinc source includes zinc sulfate, zinc chloride, zinc acetate, zinc nitrate and the like, and is preferably selected from zinc chloride and zinc nitrate, and the use of $NH_3$ or NaOH aqueous solution as the mineralization material is included. The chlorine source aqueous solution and the zinc source aqueous solution are preferably reacted at a concentration ratio (molar ratio) of chlorine to zinc of approximately 2:5, and the concentration of the zinc source aqueous solution is preferably in a range of from 0.1 to 1 M. The reaction is preferably performed at 40°C or lower, and more preferably at 25°C. The obtained zinc chloride hydroxide hydrate having Simonkolleite as a main component is a mixture of a reaction product obtained by the above-mentioned precipitate generation reaction of an aqueous zinc salt solution and an aqueous alkali solution, raw materials as unreacted products, by-products, and impurities contaminated from raw materials.

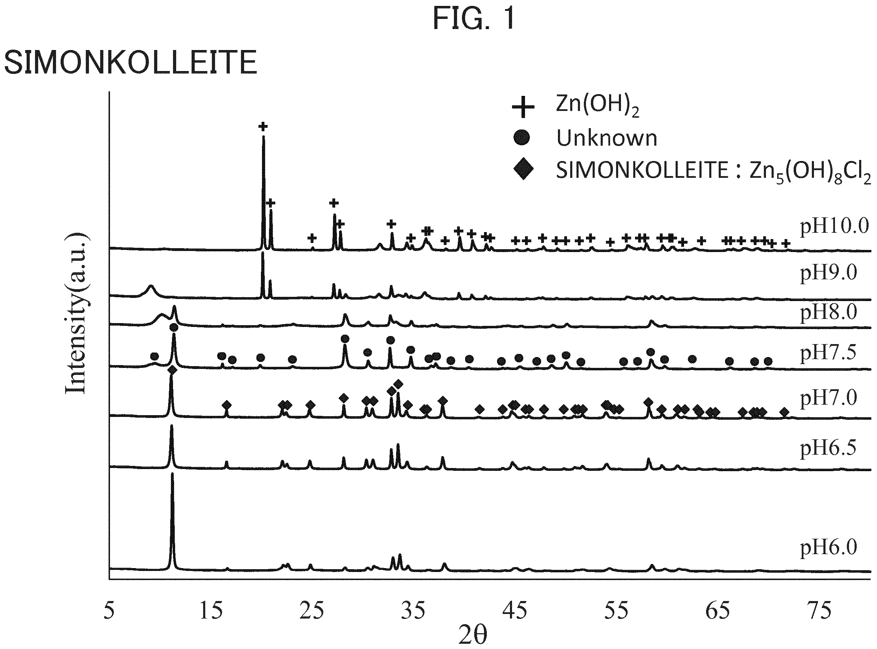

FIG. 1 shows an XRD chart of simonkolleite manufactured with varying pH conditions according to the method of manufacturing a Preparation Example of the invention. As for the XRD device, D8 ADVANCE available from Bruker Corporation is used.

As shown in the cases of pH 6.0 to 7.0 at synthesis in the chart in FIG. 1, at the XRD diffraction peaks of the zinc chloride hydroxide hydrate containing simonkolleite, the structure of $Zn_5Cl_2(OH)_8 \cdot nH_2O$ being simonkolleite is dominant, where a axis is preferably from 6.3 to 6.345 and c axis is preferably from 23.4 to 23.7. Within this range of the XRD diffraction peaks, the crystal exhibits a good effect as a therapeutic agent for a skin wound or skin roughness. At pH 7.5 or greater, the unknown peaks are dominant. The manufacturing conditions under which simonkolleite can be obtained as a main component will vary depending on the type and concentration of the chloride source and zinc source used, and an optimal condition can be found by varying the pH conditions for manufacturing. Here, the main component refers to the most abundant component in the mixture, and it is preferably not less than 60 mass%, more preferably not less than 80 mass%, and further preferably not less than 95 mass%.

To date, a large number of studies have been conducted by applying zinc compounds such as $ZnSO_4$, $ZnCl_2$, and ZnO to wound sites created in experimental animals to assess wound healing effects. These compounds are substances that supply $Zn^{2+}$ ions to the wound site. However, it has been reported that there is an optimal concentration of $Zn^{2+}$ ions for wound healing effects owing to $Zn^{2+}$ ions. At a concentration of no more than 500 $\mu$mol/L, no toxicity is shown against fibroblasts; however, the presence of high level of zinc ions (not less than 15 mmol/L) is known to increase inflammatory cell infiltration of skin and to significantly delay re-epithelialization.

In addition, pH of a body fluid having saline as a main component is approximately 7.4 to 7.5, and when the zinc chloride hydroxide of the invention having simonkolleite as a main component is dissolved in saline, variation of pH that corresponds to generation and dissolution of $Zn^{2+}$ ions is expected. Furthermore, by deciding on the following 2 states, i.e., the pH environment for supplying $Zn^{2+}$ ions and/or $Cl^-$ ions and the supply of $OH^-$ ions for efficiently activating the matrix metalloprotease (MMPs) enzymes that decompose substrate proteins via $OH^-$ of water molecules, the treatment is further promoted, and tissues that interfere with the treatment, such as scab, would not be formed, the scar remaining is prevented, and thus, the patient's QOL (Quality of Life) can be expected to improve.

In the wound healing process, it is known that active proliferation and migration of cells occurs. When cells migrate into or between tissues, the existing extracellular matrix has to be locally disrupted. A new extracellular matrix is formed simultaneously to reconstruct the tissue at the wound site. Various proteolytic enzymes are involved in these processes.

The active balance of matrix metalloproteases (MMPs) and tissue inhibitory metalloproteases (TIMPs) is responsible for both normal and pathological events, such as wound healing, tissue repair, angiogenesis, infiltration, tumor formation, and metastasis.

MMP is an enzyme that decomposes extracellular collagen and is synthesized by cells that are present between connective tissues. MMP has a zinc ion in the center and there is a $Zn^{2+}$ ion binding site at the active site. Epidermal regeneration is achieved by the epidermis cells migrating from wound edges and skin appendages (hair root, sweat glands, etc.). Thus, binding of $Zn^{2+}$ ions from the zinc compound with MMPs causes destruction of the extracellular matrix and promotes epidermal cell migration.

TIMP is an enzyme that is produced by fibroblasts, endothelial cells, and the like, and has an inhibitory effect against MMP. MMPs and TIMPs form a complex in a ratio of 1:1, and thus the collagen decomposition of MMPs is inhibited. This mechanism can facilitate fiber formation at the wound site by inhibiting specific cleavage of the helix site of the type I, II and III collagens caused by MMPs, and thus, can increase the amount of collagen in the regenerated tissue.

As stated above, it is believed that the zinc chloride hydroxide of the invention can promote cell migration by appropriately supplying zinc ions and/or chloride ions to the wound site, and can increase collagen accumulation and promote wound healing. Furthermore, by focusing on a pH environment for supplying zinc ions and/or chloride ions, the treatment is further promoted, and tissues that interfere with the treatment, such as scab, would not be formed, the scar remaining is prevented, and thus, the patient's QOL (Quality of Life) can be expected to improve.

The pharmaceutical therapeutic agent for a skin wound or skin roughness using the inorganic composition of the invention is not limited but can treat skin wounds or skin roughness. Here, the skin wound or skin roughness is a skin wound or skin roughness which is a loss in epidermis and dermis (of full thickness skin) reaching up to dermis via epidermis, or pressure ulcers, skin wound, or skin roughness which reaches up to peritoneum via epidermis and dermis.

3. Other Applications

(1) Pharmaceuticals for use with wound covering materials

The zinc chloride hydroxide having excellent zinc ion sustained-release properties of the invention that can be used as an active ingredient of pharmaceuticals is effective, as pharmaceuticals, for the healing of a skin wound or skin roughness which reaches up to dermis via epidermis, and of pressure ulcers which reaches up to peritoneum via epidermis and dermis. It is effective for the healing of skin wound or skin roughness which is a loss of full thickness skin reaching up to dermis via epidermis.

The pharmaceutical using the invention can be a therapeutic medical device for a skin wound or skin roughness, which is applied to a skin wound or skin roughness and retains the skin wound or skin roughness in a closed environment when used together with a wound covering material. Here, "applied to a skin wound or skin roughness" means that it may be applied directly to the skin wound or skin roughness, or may be applied to the skin around the skin wound or skin roughness. A wound covering material is a medical device that retains the skin wound or skin roughness in a closed environment.

Healing of skin wounds or skin roughness may involve healing in a dry environment and in a moist environment. When healing in a dry environment, generally the wound or skin roughness is covered with a breathable wound covering material for protection. Gauze, bandages, and breathable film-like wound covering materials may be used.

The therapeutic agent for a skin wound or skin roughness can be brought in a closed environment with the wound covering material and be also retained in a suitable moist environment, thereby being therapeutically effective. When the zinc chloride hydroxide of the invention which is an inorganic material is combined with the wound covering material which is an organic material, a large synergistic effect can be obtained by hybridizing the organic and inorganic materials.

When the active ingredient of the invention is used as a pharmaceutical, saline or the like is used as a solvent to make a liquid drug for use in the form of, for example, lotion, solution, cream, ointment, or spray. Furthermore, the active ingredient can also be used in a dosage form of powder body such as powder or of ointment when exudate is present in the wound and the wound is moist. If necessary, it can be made into a thickening state or gel agent (sometimes referred to as a gel) which is an intermediate state of the above. The therapeutic agent for a skin wound or skin roughness using the zinc chloride hydroxide of the invention is therapeutically effective in a moist environment as the therapeutic agent can be brought in a closed environment with the wound covering material and its dosage form can be adjusted into a liquid or powder so as to maintain the individual wound in a suitable moist state. Regardless of whether the dosage form is liquid or powder, when the pharmaceutical therapeutic agent is used with the wound covering material, the tissues, such as scab, that interfere with the treatment would not be formed, and the scar remaining is prevented so that a high QOL (Quality of life) of a patient is obtained.

<Medical Device Comprising Therapeutic Agent and Wound Covering Material>

In another aspect, the therapeutic agent of the invention may be carried in the covering material in the manufacture of the wound covering material so as to be used in a medical device. This may be a medical device in which the therapeutic agent of the invention is applied to, contained in, or adhered to the wound covering material and the wound covering material retains the skin wound or the skin roughness in a closed environment. The therapeutic agent is contained and deposited when the wound covering material is synthesized.

Alternatively, the therapeutic agent may be fixed by spraying, immersing, or applying the therapeutic agent to the application side surface of the wound covering material that has been processed into a sheet form, and then used as a medical device.

<Wound Covering Material>

The wound covering material according to the invention is a chitin wound covering material.

The chitin would covering material is obtained by removing calcium or proteins which become an allergen from a shell of a crustacean and purifying the resultant to obtain amino polysaccharides, which are formed into a sheet form. This has a high bioaffinity, and analgesic and hemostatic effects can be expected. While the material has an excellent water absorption and allows the retention of moist environments, secondary dressing materials are needed. The chitin processed into a cotton form is thick, and when a large amount of exudate is present, it is replaced daily, and when the amount is reduced, the time till replacement is extended. A gauze coated with the sponge-like processed chitin is also useful.

The therapeutic agents of the invention are pharmaceuticals sets with these wound covering materials. The form of use (treatment technique) as a pharmaceutical at this time is as follows.

The active ingredient of the invention may be a therapeutic agent for a skin wound or skin roughness as a pharmaceutical, and may include a pharmaceutically acceptable carrier, if necessary. Examples of the carrier include organic powders, inorganic powders, organic solvents and inorganic solvents, more specifically, corn starch, cereal flour, talc, water, saline, alcohols, polyhydric alcohols, or mixtures thereof. To have a form of the pharmaceutical, a thickening agent or the like may be added, and the ingredient is processed into a gel form or a paste form having the improved handleability.

<Carriers Used in Therapeutic agent>

Examples of the carrier include hydrocarbons such as alpha-olefin oligomers, paraffin waxes, ceresin, microcrystalline wax, animal and vegetable oils such as persic oil, olive oil, beef fat, and mink oil, synthetic esters such as cetyl octanoate, isopropyl myristate, and cetyl palmitate, natural animal and vegetable waxes such as jojoba oil, carnauba wax, candelilla wax, Japan wax, and beeswax, silicone oils and derivatives thereof such as sorbitan stearate, polyoxyethylene glyceryl tristearate, polyoxyethylene lauryl ether, decaglyceryl trioleate, sucrose monolaurate ester, dimethylpolysiloxane, and methylphenyl polysiloxane, and the like.

Fluororesins such as perfluoropolyether, alcohols such as ethanol, 1,3-butylene glycol, propylene glycol, and diglycerin; carrageenan, xanthan gum, sodium carboxymethylcellulose, collagen, elastin, silk, cellulose, lactoferrin and other proteins and hydrolysates thereof, powders of anhydrous silicic acid, nylon powder, polyalkyl acrylate, alumina, and iron oxide may be used.

In addition, ultraviolet absorbers, vitamins, ureas, dried-seawater products, anti-inflammatory agents, amino acids and derivatives thereof, lecithin, colorants, perfumes, preservatives, and the like, oils such as egg yolk oil, macadamia nut oil, cottonseed oil, avocado oil, coconut oil, palm oil, palm kernel oil, corn oil, peanut oil, beef fat, and carnauba wax can be used.

Still others include beeswax, liquid paraffin, lanolin, squalane, stearic acid, laurate esters, myristic acid esters, isostearyl alcohol, purified water, electrolyzed water, ethyl alcohol and the like. That is, in general, those commonly blended in cosmetic products and quasi drugs can be used as the carrier in the therapeutic agent for a skin wound or skin roughness of the invention. The carrier may not be used.

For example, other components that may be added to the therapeutic agent for a skin wound or skin roughness of the invention are selected depending on those to be actually added in cosmetics or quasi drug. Although it cannot be strictly distinguished, humectants include glycerin, sorbitol, polyethylene glycol, pyrrolidone carboxylic acid and salts thereof, collagen, 1,3-butylene glycol, hyaluronic acid and salts thereof, chondroitin sulfate and salts thereof, xanthan gum, and the like.

Antioxidants include ascorbic acid, $\alpha$-tocopherol, dibutylhydroxytoluene, parahydroxyanisole, and the like. Surfactants include sodium stearyl sulfate, diethanolamine cetyl sulfate, polyethylene glycol monostearate, ethylene glycol monostearate, polyoxyethylene hydrogenated castor oil, soybean lysophospholipid solution, polyoxyethylene sorbitan monooleate, and the like.

Preservatives include inorganic pigments such as phenoxyethanol, ethylparaben, butylparaben, zinc oxide, and the like.

Anti-inflammatory agents include glycyrrhizinic acid derivatives, salicylic acid derivatives, hinokitiol, zinc oxide, allantoin, and the like.

Whitening agents include placenta extract, glutathione, saxifraga extract, ascorbic acid derivatives, arbutin, and the like.

Blood circulation promoters include $\gamma$-oryzanol, sodium dextran sulfate, and the like.

Antiseborrheic agents include sulfur, thianthol, and the like.

Examples of thickening agents include carboxyvinyl polymers and the like.

pH adjusting agents include lactic acid, citric acid, malic acid, glycolic acid, sodium hydroxide, hydrotalcite, and the like.

(2) Effect as a pharmaceutical for use with wound covering materials

Zinc chloride as a pharmaceutical product of the zinc chloride hydroxide of the invention is free of a drying action or antimicrobial properties in contrast with zinc oxide and is useful as an active ingredient of pharmaceuticals. Also, it is useful as additives to other materials. Specifically, it can be used as an additive to a pharmaceutical composition as well as an additive to cosmetics and can exert different, synergistic, or enhanced effects on the substances to which the additives were added.

Examples

The invention will be described in details using the examples below, but the invention is not limited thereto.

(Preparation Example 1)

500 mL of 0.08 M aqueous ammonium chloride solution was prepared in a reaction vessel, and 1000 mL of 0.1 M aqueous zinc chloride solution was separately prepared as a drip reaction solution. 30 mass% aqueous sodium hydroxide solution was prepared as a pH adjusting solution.

Using a pH controller to which a pump was connected, the aqueous zinc chloride solution and aqueous sodium hydroxide solution were added dropwise with the above-mentioned aqueous ammonium chloride solution being maintained at pH 6.5 under stirring. After all of the aqueous zinc chloride solution was added, the reaction solution was stirred for 16 hours and was allowed to stand.

After that, the reaction solution was solid-liquid separated by centrifugation, and the obtained solid was washed with ; centrifugation was repeated three times. The washed precipitate was vacuum dried to obtain dry powder of simonkolleite having the composition range shown in Formula (1) .

(Preparation Example 2)

pH at synthesis was set to 5.5 to 10, and various samples were prepared by the same process as in Preparation Example 1 while varying the pH at the time of preparation including that of Preparation Example 1 (pH 6.5). At pH 5.5, since the pH was too low, no precipitate was obtained.

(Dissolution Test)

A dissolution test using 30 g of saline for 0.6 g of each dry powder obtained in Preparation Example 2 was performed by stirring method, and the amount of dissolved $Zn^{2+}$ ions and pH were measured. In the method of the dissolution test, the BET specific surface area of the simonkolleite-containing zinc chloride hydroxide hydrate was adjusted to from 10 to 150 $m^2/g$, the mass ratio of the simonkolleite-containing zinc chloride hydroxide hydrate to the saline was 1:50, and the time for stirring using a rotor at 500 rpm was set to be 3 hours. The pH and the amount of dissolved

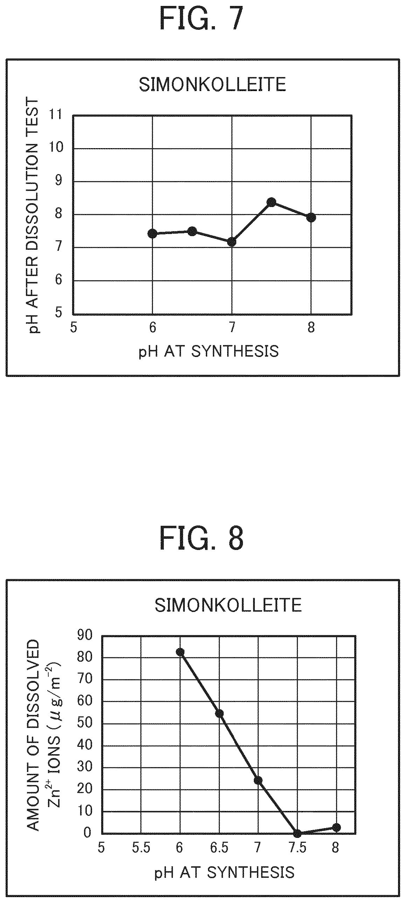

$Zn^{2+}$ ions after the dissolution test were measured. The results are shown in FIGS. 7 and 8, and Table 1.

As shown in FIG. 8, the pH around 7.5 is considered to be a specific point in the synthesis of the simonkolleite-containing zinc chloride hydroxide hydrate, and an improved yield and crystallinity of simonkolleite can be observed at pH of less than 7.5. On the other hand, it is believed that a heterogenous phase occurs at pH of not less than 7.5, and zinc hydroxide is predominantly synthesized at pH of not less than 8.5.

[Table 1]

| pH at synthesis | 5.5 | 6 | 6.5 | 7 | 7.5 | 8 |
|---|---|---|---|---|---|---|
| pH after dissolution test | - | 7.42 | 7.49 | 7.17 | 8.37 | 7.91 |
| Amount of dissolved $Zn^{2+}$ ions after dissolution test ($\mu g/m^2$) | - | 82.7 | 54.6 | 24.2 | 0.08 | 2.91 |

(Pharmaceutical Example 1)

To an approximately 500 g SD rat, 0.2 mL/500g of 2% Sederac (xylazine) was administered by intramuscular injection and the rat was sedated, and then, 2% of sevoflurane inhalation anesthetic was used to carry out a general anesthesia. Following local anesthesia by administering xylocaine (lidocaine + 2% adrenaline) to the rat ventral side, a wound with all thickness skin loss of a diameter of 10 mm from epidermis reaching up to subcutaneous tissue was created, and 0.01 g of the powder of the above-mentioned Preparation Example 1 was applied to the wound created part wound with all thickness skin loss, and further covered with Duoactive which is a medical wound covering material not falling within the scope of the present claims.

(Tissue Staining and Hematoxylin-Eosin (H-E) Staining)

The method of tissue staining was carried out as follows: wound site trimming (excision) → formalin fixation → degreasing treatment (immersed in xylene for 24 hours) → dehydration treatment.

The dehydration treatment was carried out as follows: samples were immersed in 70% ethanol for 12 hours and the ethanol was removed by volatilization, and then the samples were dehydrated by 80% ethanol, 90% ethanol, and 95.5% ethanol for 30 minutes each, and washed twice with xylene.

The samples were then embedded in paraffin → sections were prepared → H-E staining was carried out.

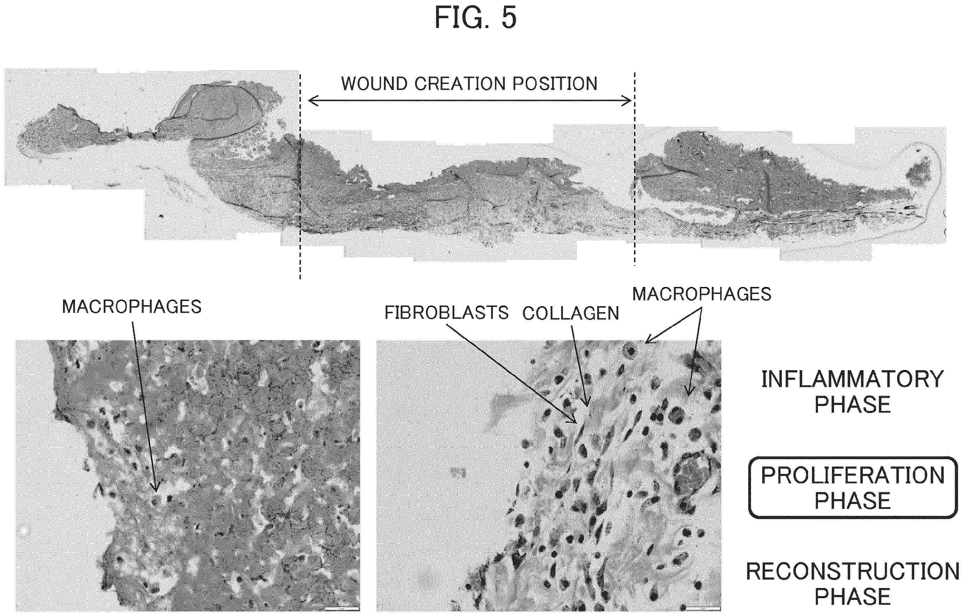

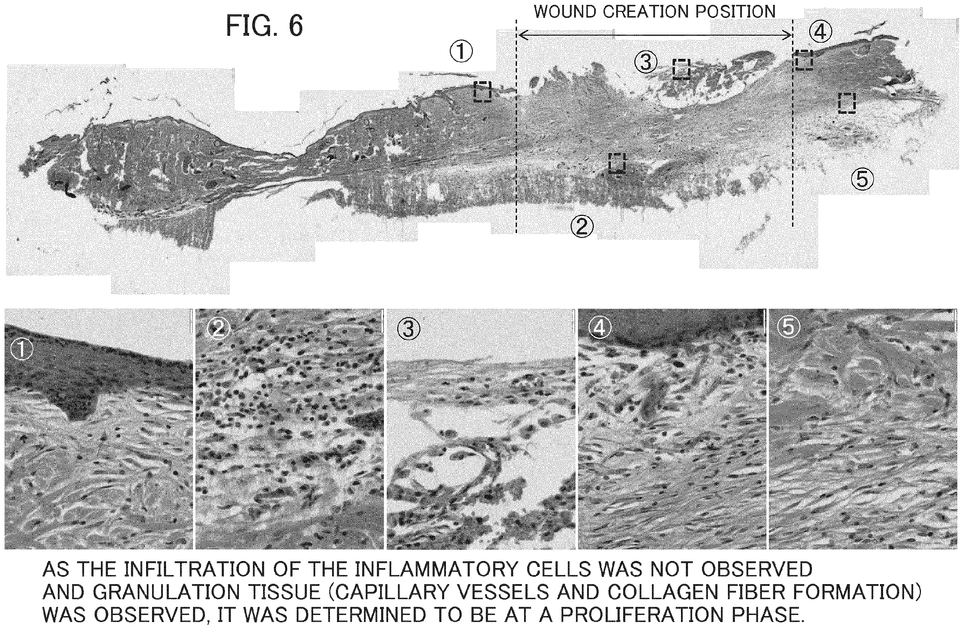

The cell nucleus was stained with hematoxylin in a blue-violet color and the cytoplasm, collagen fibers, and muscle fibers were stained in red color with eosin. → The sample after staining was enclosed in a prepared slide, and the microscopical observation results are shown in FIGS. 5 and 6.

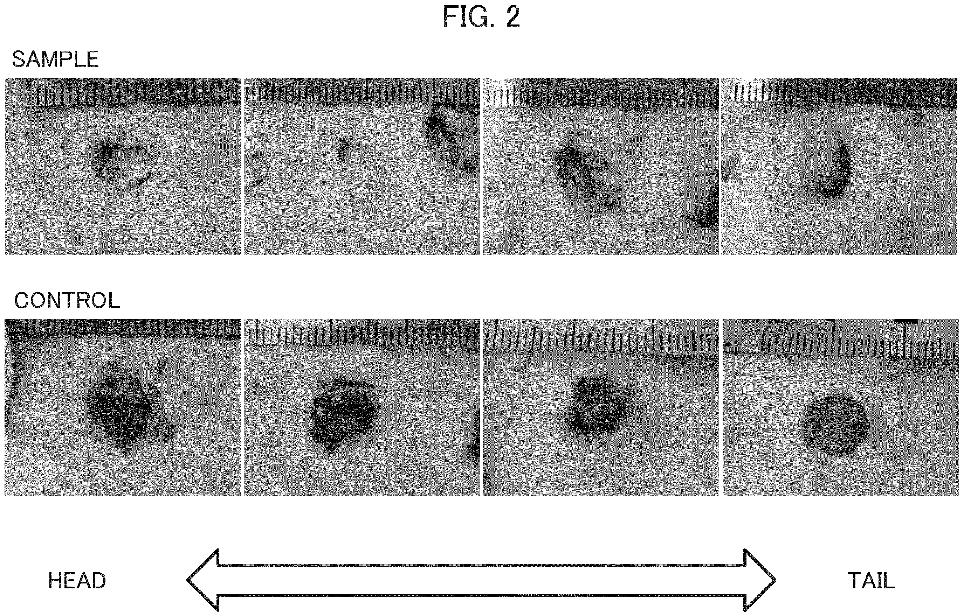

Observation results of the healing site after one week of covering with the wound covering material (FIG. 2): The therapeutic agent obtained in Preparation Example 1 was applied to the sample and then the sample was covered with the wound covering material. After one week, a white tissue was observed in the wound site of the sample; however, in a control which was only covered with Duoactive, a medical wound covering material not falling within the scope of the present claims, without using the therapeutic agent, the peritoneum was clearly seen, and the wound was not healed.

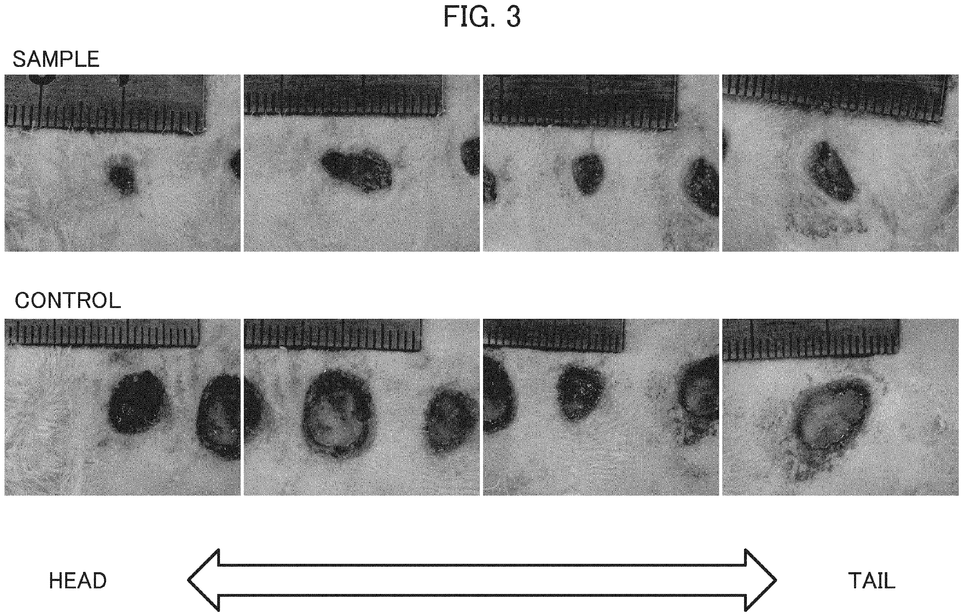

Observation results of the healing site after 2 weeks of covering with the wound covering material (FIG. 3): The therapeutic agent obtained in Preparation Example 1 was applied to the sample and then the sample was covered with the wound covering material. In the sample after 2 weeks, an obvious reduction of the wound site was observed and the peritoneum was not recognized. On the other hand, in the control where the therapeutic agent was not used, while a slight reduction in the wound site was observed, an unhealed site in which peritoneum is recognized was also observed.

(Measurement Method of Re-epithelialization Rate)

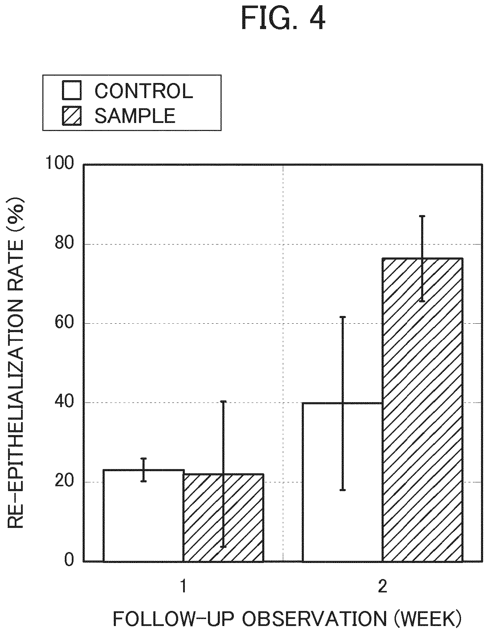

Magnified photographs of the skins were taken at the time of wound creation and after treatment. The initial wound site at the time of wound creation was measured and drawn into the photographs after 1 week and 2 weeks with a solid line. The area of the initial wound site $W_0$ was measured, the area of the unhealed site (Wt) after treatment was measured by Image J [open source published on the web; Wayne Rasband (NIH)], and the rate of re-epithelialization is calculated in % from the measured area using the following equation. The rates of re-epithelialization after treatment shown in FIG. 2 and FIG. 3 are shown in Table 2 and FIG. 4 together with those of the controls.

$$\text{Re-epithelialization rate (\%)} = (W_0 - W_t)/W_0 \times 100\%$$

[Table 2]

| Follow-up observation Time (week) | Comparative Examples | Examples of the invention |
|---|---|---|
| 1 | 23.08 | 22.04 |
| 2 | 39.88 | 76.38 |

Based on the results shown in Table 2 and FIG. 4, the rate of re-epithelialization of the Pharmaceutical Example of the invention (sample) after 2 weeks was about 1.9 times that of the Comparative Example (control).

Re-epithelialization rate: a t-test was performed on data of two groups of Comparative Example and Pharmaceutical Examples of the invention after 2 weeks having different variances of re-epithelialization rate. The t-test was performed on three out of four wounds and no significant difference was observed due to a relatively large error bar; however, a significant difference was observed in the Pharmaceutical Examples of the invention in a case of calculation from the four wounds. In the calculation of the re-epithelialization rate, the white tissue of the control was evaluated as an unhealed region.

The calculation from the four wounds is shown in Table 3.

[Table 3]

| | Comparative Examples | Examples of the invention |
|---|---|---|
| Number of examples | 4 | 4 |
| Average | 28.62 | 74.47 |
| Variance | 823.21 | 91.02 |
| Standard Deviation | 28.69 | 9.54 |
| Standard Error | 14.35 | 4.77 |
| Mean difference | -45.84 | |
| Degree of freedom | 3 | |
| t-value | -3.03 | |
| p-value in t-test | 0.0435 | |

FIGS. 5 and 6 are micrographs of skin cross sections showing histological observation results after 1 week and after 2 weeks. The top views of FIGS. 5 and 6 are overall views at a magnification of 36 times, and the bottom views of FIGS. 5 and 6 are enlarged views, at a magnification of 360 times, of the part shown in the top views.

FIG. 5 shows in its top view the healing state of the wound created part after one week and in its bottom view the enlarged view of the same. FIG. 6 shows in its top view wound created parts 2 and 3 and its bottom view micrographs showing histological assessments of the wound created part 2 and the wound created part 3, namely the wound created parts shown in the top view, after 2 weeks. Micrographs showing histological assessments of part 1, part 4, and part 5, other than the wound created parts, were shown together, and the progress of the wound healing was compared to the normal skin condition.

[Tissue observation results after 1 week (FIG. 5)]:

When the therapeutic agent of the invention was applied, the presence of collagen, fibroblasts, or macrophages was confirmed, and therefore it was determined to be at a proliferation phase. Also, as the white tissue is believed to be a granulation tissue, if the white tissue is counted as a healed tissue in the calculation of the reepithelization rate, the therapeutic agent of the invention could be determined to exhibit a very high skin regeneration ability.

[Tissue observation results after 2 weeks (FIG. 6)]:

As the infiltration of inflammatory cells was not observed and granulation tissue (capillary vessels and collagen fiber formation) was observed, it was determined to be at a proliferation phase.

These results revealed that the therapeutic agent comprising simonkolleite of the invention is an effective wound treatment material. It is believed that skin regeneration or hair root regeneration can occur without causing inflammation. Accordingly, the therapeutic agent using the active ingredient of the invention has a therapeutic effect on a skin wound or skin roughness which does not reach up to dermis via epidermis, a therapeutic effect on a skin wound or skin roughness which reaches up to dermis via epidermis, or a therapeutic effect on a skin wound or skin roughness which is an all thickness skin loss reaching up to dermis via epidermis. Thus, the therapeutic agent using the active ingredient of the invention has not only a healing effect on the skin wound shown in the examples but also on similar wounds caused by severe roughness.

(Pharmaceutical Example 2)

A wound with all thickness skin loss was created as in Example 1, and powder of sample from Preparation Example 2 was applied to each of the created wounds with all thickness skin loss and the wounds were further covered with Duoactive which is a medical wound covering material not falling within the scope of the present claims. The re-epithelialization rate was between 80 and 90%, and the therapeutic agent of the invention, which was obtained at pH in a range of not less than 6.0 and less than 7.5 as a manufacturing condition, has an excellent healing effect.

Table 4 below shows the pH after the dissolution test and the results of evaluation on wound healing effects after 2 weeks (re-epithelialization rate, collagen regeneration, hairball regeneration, and granulation formation).

[Table 4]

| | 5.5 | 6 | 6.5 | 7 | 7.5 | 8 | - |
|---|---|---|---|---|---|---|---|
| pH at synthesis | 5.5 | 6 | 6.5 | 7 | 7.5 | 8 | - |
| pH after dissolution test | - | 7.42 | 7.49 | 7.17 | 8.37 | 7.91 | - |
| Amount of dissolved $Zn^{2+}$ ions after the dissolution test ($\mu g/m^2$) | - | 82.7 | 54.6 | 24.2 | 0.08 | 2.91 | - |
| Healing state after 2 weeks | | | | | | | Control |
| Re-epithelialization rate | - | 1.9 | 1.9 | 1.8 | 1.4 | 1.3 | 1 |
| Collagen thickness | - | ◎ | ◎ | ◎ | ○ | ○ | △ |
| Collagen Orientation | | ◎ | ◎ | ◎ | △ | △ | × |
| Hairballs | - | ◎ | ◎ | ◎ | ○ | ○△ | × |
| Granulation formation | | ◎ | ◎ | ○ | ○△ | ○△ | ○ |

The evaluation method of Table 4 is based on evaluation according to the above-described measurement method where the re-epithelialization rate of the control is regarded as 1. For the evaluation of collagen, thick collagen fibers extending in one direction were observed. With respect to hairballs, the observation results show whether the hairballs could be confirmed. For the evaluation of granulation formation, it was determined whether tissues composed of capillary vessels and fibroblasts were observed.

Collagen: For evaluation of collagen, collagen fiber thickness and orientation were assessed in comparison with healthy skin cells.

Collagen thickness : × : very thin

△: Thin
○: Slightly inferior
◎: Same as healthy skin cells

Collagen Orientation : × : Random

△: Very inferior
○: Slightly inferior
◎: Same as healthy skin cells

Hairballs: × : No formation of hairballs is observed.

∘Δ: Slight formation of hairballs.

∘: Hairballs are formed.

◎: The formation of hairballs is prominently observed.

Granulation formation: ×: No granulation is observed.

∘Δ: Slight granulation formation.

∘: Granulation is observed.

◎: Granulation is prominently observed.

The results, in which that of the control as a comparison is included, are shown in Table 4. Simonkolleite which is a pharmaceutical of the invention not only has a higher degree of re-epithelialization than the control, but also exhibits an equivalent recovery as a healthy skin tissue in the regenerated tissue, in particular, in the appearance of collagen, and thus it is found to have a great effect.

Accordingly, it is believed that the product of the invention may be applied to a commercially available organic based wound covering material, whereby the use of an inorganic based material of the active ingredient of the invention leads to hybridization of organic based/inorganic based materials, resulting in a great synergistic effect and a great improvement in the wound healing capability.

(Application Form)

The pharmaceutical examples described above take an application form of powder for use in treatment techniques in which the basic zinc salt powder of the invention is spread onto the wound part and a medical wound covering material covers the wound part. The inventors conducted diligent research on the application form (treatment technique) of the basic zinc salt powder of the invention. The application form (treatment technique) and the healing effect when used as an ointment along with a covering material will be described hereinafter.

(Ointment Preparation Example 1)

4.9 g of white vaseline was placed in a 50-mL beaker, heated to 60°C, and 0.1 g of liquid paraffin was added and mixed, after which 5 g of dry powder obtained in Preparation Example 1 was added thereto and sufficiently stirred to produce 10 g of ointment (amount of active ingredient: 50 mass%). The ointment was applied to the created wound with all thickness skin loss, and observation of wound appearance after 2 weeks and tissue observation of the wound part were carried out. The results are shown in Table 5.

(Comparative Example 1, using only an ointment base as a control)

Only the ointment base of white vaseline and paraffin which does not contain the active ingredient was applied as a comparative example to the wound with all thickness skin loss created as in the above-mentioned Ointment Preparation Example, and observation of wound appearance after 2 weeks and tissue observation of the wound part were carried out as a control with the base only. The results are shown in Table 5.

(Covering material usage examples 2 and 3)

Using the powder obtained in Preparation Example 1, in Covering material usage example 2, 5 g of the dry powder of Preparation Example 1 was kneaded into 5 g of hydrogel (active ingredient: 50 mass%), and in Covering material usage example 3, the powder of Preparation Example 1 was fixed onto a hydrogel surface (wound contacting side) by a shot blasting method in an amount of 40 mass% (4 g of dry powder for 6 g of hydrogel), whereby Covering material usage example 2 and Covering material usage example 3 were prepared, respectively. Here, the hydrogels used are those used in the commercially available medical wound covering material Duoactive which does not fall within the scope of the present claims. Covering material usage example 2 and Covering material usage example 3 were each applied to the wound with all thickness skin loss as created in the above-mentioned Ointment Preparation Example, and observation of wound appearance after 2 weeks and tissue observation of the wound part were carried out. Results are shown in Table 5.

(Comparative Example 2, using only the covering material as a control)

A control (comparative example) in which the created wound with all thickness skin loss was merely covered with Duoactive was prepared, and observation of wound appearance after 2 weeks and tissue observation of the wound part were carried out. Results are shown in Table 5.

The Ointment Preparation Example 1, Covering material usage examples 2 and 3 were all evaluated for healing effect after 2 weeks (collagen thickness, collagen orientation, hairball regeneration, and granulation formation) and the results are shown. The evaluation criteria are the same as in Table 4.

The Simonkolleite which can be utilized as a pharmaceutical or medical device of the invention in Ointment Preparation Example 1 and Covering material usage examples 2 and 3 achieves recovery of the regenerated tissues to the same level as of a normal skin tissue, in particular, in terms of the appearance of collagen, and thus exhibits great effect, which is equivalent to the case the powder application in Pharmaceutical Example 1 and Pharmaceutical Example 2. The simonkolleite is also highly effective from the perspective of convenience at the clinical site.

[Table 5]

|  | Ointment Preparation Example 1 | Control only using base | Covering material usage example 2 | Covering material usage example 3 | Control only using covering material |
|---|---|---|---|---|---|
| Collagen thickness | ◎ | Δ | ◎ | ◎ | Δ |
| Collagen Orientation | ◎ | × | ◎ | ◎ | × |
| Hairballs | ◎ | × | ◎ | ◎ | × |
| Granulation formation | ◎ | ○Δ | ◎ | ◎ | ○ |

(Evaluation of test results)

The various test results described above can be summarized as described below. Since the wound healing agent causes an inflammatory response in wound healing when the pH value is 8 or greater, the pH of the wound healing agent is suitably in a range of neutral to 8. In view of the pH after the dissolution test in comparison to the pH at synthesis (FIGS. 7 and 8), the pH at synthesis exceeding an optimal pH is only 7.5, and the amount of dissolved $Zn^{2+}$ ions that play an important role in the wound healing process is also small. This result shows that, when the pH is 7.5 at synthesis, $Zn^{2+}$ ions do not bind to the active sites of MMPs, thus no destruction of the extracellular matrix occurs, and therefore cell migration in the wound healing process is not promoted, resulting in no promotion of wound healing. When the pH at synthesis is other than the region of around 7.5, it is within the optimal pH range, and as the amount of dissolved $Zn^{2+}$ ions is large, the wound healing is considerably promoted. As shown in Table 4, the wound healing assessment on rats meets the above consideration. It is experimentally revealed that particularly the pH 7 at synthesis, at which the amount of dissolved zinc ions is large, is within the optimal pH range, and the wound healing is remarkable. As a reason for the dissolution behavior showing the specific behavior in a region around pH 7.5 at synthesis, it is assumed that a transient phase exists between a state where the yield of simonkolleite as a single phase simply increases until pH 7 and a state where a hydroxide phase such as $Zn(OH)_2$ other than simonkolleite is dominant at pH 8 or greater; however, the details are not clear at the moment.

Industrial Applicability

The zinc chloride hydroxide having excellent zinc ion sustained-release properties of the invention allows an appropriate sustained-release of zinc ions at a suitable condition when used as a pharmaceutical, and it can be used as an active ingredient of pharmaceuticals. The zinc chloride hydroxide of the invention as an active ingredient of pharmaceuticals has an excellent stability, and the manufacturing method thereof is simple; therefore, the zinc chloride hydroxide is industrially useful.

**Claims**

1. A kit-of-parts comprising a therapeutic agent for a skin wound or skin roughness and a wound covering material for retaining the therapeutic agent and the skin wound or skin roughness in a closed environment,

wherein the therapeutic agent comprises a zinc chloride hydroxide as an active ingredient, wherein the zinc chloride hydroxide is represented by Formula (1) below,

$$Zn_{4-6}Cl_{1-3}(OH)_{7-8} \cdot nH_2O \qquad (1)$$

where n is 0 to 6, and
wherein the wound covering material is a chitin wound covering material.

2. The kit-of-parts according to claim 1, wherein the therapeutic agent is dissolved in saline, a ratio of the therapeutic agent to saline is from 0.1 g/L to 100 g/L, the therapeutic agent is represented by Formula (1) above, when n = 0 (anhydrous), a zinc concentration relative to a total amount of the therapeutic agent is from 45 mass% to 75 mass% as metal zinc, and a zinc concentration in the saline aqueous solution of the therapeutic agent is from 0.045 g/L to 75 g/L.

3. The kit-of-parts according to claim 1 or 2, wherein the therapeutic agent is in the form of powder, lotion, solution, cream, ointment, spray, or gel, and is configured to be applied or sprayed to a part of a skin wound or skin roughness to be utilized.

4. The kit-of-parts according to any one of claims 1 to 3, wherein the therapeutic agent for a skin wound or skin roughness is present by being applied to, contained in, or adhered to the wound covering material.

5. The kit-of-parts according to claim 4, wherein the wound covering material is manufactured to carry the therapeutic agent , or the therapeutic agent is fixed through spray, immersion, or application to an application side surface of the wound covering material that has been processed into a sheet form.

6. The kit-of-parts according to any one of claims 1 to 5, for use in a method of treating a full-thickness skin wound or skin roughness, or for treating full-thickness peritoneal pressure ulcers.

7. A method of manufacturing the zinc chloride hydroxide specified in claim 1, comprising maintaining an almost constant pH by acid or alkali in the presence of aqueous solution including chloride ions, continuously supplying zinc ions from an aqueous zinc solution, and precipitating zinc chloride hydroxide having a main component of simonkolleite, wherein the pH is not less than 6.0 and less than 7.5, and a temperature is lower than 40°C.

8. The method of manufacturing the zinc chloride hydroxide according to claim 7, wherein the zinc ion donor is at least one selected from the group consisting of zinc chloride and zinc nitrate.

9. The method of manufacturing the zinc chloride hydroxide according to claim 7 or 8, wherein the chloride ion donor is at least one selected from the group consisting of ammonium chloride and sodium chloride.

**Patentansprüche**

1. Kombinationsprodukt, das ein therapeutisches Mittel für eine Hautwunde oder Hautrauheit und ein Wundabdeckungsmaterial für das Halten des therapeutischen Mittels und der Hautwunde oder Hautrauheit in einer geschlossenen Umgebung umfasst,

wobei das therapeutische Mittel ein Zinkchloridhydroxid als einen Wirkstoff umfasst, wobei das Zinkchloridhydroxid durch die folgende Formel (1) wiedergegeben wird:

$$Zn_{4-6}CL_{1-3}(OH)_{7-8} \cdot nH_2O \qquad (1)$$

wobei n zwischen 0 und 6 ist, und
wobei das Wundabdeckungsmaterial ein Chitin-Wundabdeckungsmaterial ist.

2. Kombinationsprodukt nach Anspruch 1, wobei das therapeutische Mittel in einer Salzlösung aufgelöst ist, das Verhältnis des therapeutischen Mittels zu der Salzlösung zwischen 0,1 g/L bis 100 g/L beträgt, das therapeutische Mittel durch die oben genannte Formel (1) wiedergegeben wird, wenn n = 0 (anhydrisch), die Zinkkonzentration relativ zu der Gesamtgröße des therapeutischen Mittels zwischen 45 Massenprozent und 75 Massenprozent als Metallzink beträgt, und die Zinkkonzentration in der wässrigen Salzlösung des therapeutischen Mittels zwischen 0,045 g/l und 75 g/l beträgt.

3. Kombinationsprodukt nach Anspruch 1 oder 2, wobei das therapeutische Mittel die Form eines Pulvers, einer Lotion, einer Lösung, einer Creme, einer Salbe, eines Sprays oder eines Gels aufweist und konfiguriert ist, um für eine Nutzung auf einen Teil einer Hautwunde oder Hautrauheit aufgetragen oder gesprüht zu werden.

4. Kombinationsprodukt nach einem der Ansprüche 1 bis 3, wobei das therapeutische Mittel für eine Hautwunde oder Hautrauheit vorgesehen ist, indem es auf das Wundabdeckungsmaterial aufgetragen, in diesem enthalten oder an dieses geklebt ist.

5. Kombinationsprodukt nach Anspruch 4, wobei das Wundabdeckungsmaterial hergestellt ist, um das therapeutische Mittel zu tragen, oder das therapeutische Mittel durch Sprühen, Eintauchen oder Auftragen an einer Anwendungs-seitenfläche des Wundabdeckungsmaterials, das zu einer Blattform verarbeitet wurde, fixiert ist.

6. Kombinationsprodukt nach einem der Ansprüche 1 bis 5 für die Verwendung in einem Verfahren zum Behandeln einer größeren Hautwunde oder Hautrauheit oder zum Behandeln von größeren peritonealen Druckgeschwüren.

7. Verfahren zum Herstellen des in Anspruch 1 genannten Zinkchloridhydroxids, das das Aufrechterhalten eines beinahe konstanten pH-Werts durch eine Säure oder Base in Anwesenheit einer wässrigen Lösung mit darin enthaltenen Chloridionen, das kontinuierliche Zuführen von Zinkionen aus einer wässrigen Zinklösung und das Ausfällen von Zinkchloridhydroxid mit einer Hauptkomponente von Simonkolleit umfasst, wobei der pH-Wert nicht kleiner als 6,0 und kleiner als 7,5 ist und die Temperatur niedriger als 40°C ist.

8. Verfahren zum Herstellen des Zinkchloridhydroxids nach Anspruch 7, wobei der Zinkionenspender wenigstens ein aus der Gruppe, die aus Zinkchlorid und Zinknitrat besteht, ausgewählter ist.

9. Verfahren zum Herstellen des Zinkchloridhydroxids nach Anspruch 7 oder 8, wobei der Chloridionenspender wenigstens ein aus der Gruppe, die aus Ammoniumchlorid und Natriumchlorid besteht, ausgewählter ist.

**Revendications**

1. Kit de composants comprenant un agent thérapeutique pour une lésion de peau ou une rugosité de peau et matériau de recouvrement de plaie pour maintenir l'agent thérapeutique et la lésion de peau ou la rugosité de peau dans un environnement fermé,

l'agent thérapeutique comprenant un hydroxyde de chlorure de zinc comme ingrédient actif, l'hydroxyde de chlorure de zinc étant représenté par la Formule (1) ci-dessous,

$$Zn_{4\text{-}6}Cl_{1\text{-}3} (OH)_{7\text{-}8} \cdot nH_2O \qquad (1)$$

où n a la valeur de 0 à 6, et
le matériau de recouvrement de plaie est un matériau de recouvrement de plaie à base de chitine.

2. Kit de composants selon la revendication 1, l'agent thérapeutique étant dissous dans une solution saline, un rapport de l'agent thérapeutique à la solution saline étant de 0,1 g/l à 100 g/l, l'agent thérapeutique étant représenté par la Formule (1) ci-dessus, lorsque n = 0 (anhydre), une concentration en zinc par rapport à une quantité totale de l'agent thérapeutique étant de 45 % en masse à 75 % en masse sous la forme de zinc métal, et une concentration en zinc dans la solution saline aqueuse de l'agent thérapeutique étant de 0,045 g/l à 75 g/l.

3. Kit de composants selon la revendication 1 ou 2, l'agent thérapeutique se présentant sous la forme d'une poudre, d'une lotion, d'une solution, d'une crème, d'un onguent, d'une pulvérisation, ou d'un gel, et étant conçu pour être appliqué ou pulvérisé à une partie d'une lésion de la peau ou à une rugosité de peau à utiliser.

4. Kit de composants selon l'une quelconque des revendications 1 à 3, l'agent thérapeutique pour une lésion de la peau ou une rugosité de peau étant présent en étant appliqué au, contenu dans, ou adhéré au matériau de recouvrement de plaie.

**5.** Kit de composants selon la revendication 4, le matériau de recouvrement de plaie étant fabriqué pour supporter l'agent thérapeutique, ou l'agent thérapeutique étant fixé à travers une pulvérisation, une immersion, ou une application à une surface latérale d'application du matériau de recouvrement de plaie qui a été transformé en une forme de feuille.

**6.** Kit de composants selon l'une quelconque des revendications 1 à 5, pour l'utilisation dans un procédé de traitement d'une lésion de la peau ou d'une rugosité de peau de pleine épaisseur, ou de traitement d'ulcères de pression péritonéale de pleine épaisseur.

**7.** Procédé de fabrication de l'hydroxyde de chlorure de zinc spécifié selon la revendication 1, comprenant le maintien d'un pH pratiquement constant par un acide ou un agent alcalin en la présence d'une solution aqueuse comprenant des ions chlorures, l'alimentation de manière continue d'ions zinc d'une solution aqueuse de zinc, et la précipitation de l'hydroxyde de chlorure de zinc ayant un constituant principal de simonkolleite, le pH n'étant pas inférieur à 6,0 et inférieur à 7,5, et une température étant inférieure à 40°C.

**8.** Procédé de fabrication de l'hydroxyde de chlorure de zinc selon la revendication 7, le donneur d'ions zinc étant au moins l'un sélectionné dans le groupe constitué du chlorure de zinc et du nitrate de zinc.

**9.** Procédé de fabrication de l'hydroxyde de chlorure de zinc selon la revendication 7 ou 8, le donneur d'ions chlorure étant au moins l'un sélectionné dans le groupe constitué du chlorure d'ammonium et du chlorure de sodium.

# FIG. 1

SIMONKOLLEITE

EP 3 552 609 B1

FIG. 2

SAMPLE

CONTROL

HEAD ⟷ TAIL

FIG. 3

# FIG. 4

FIG. 5

WOUND CREATION POSITION

INFLAMMATORY PHASE

PROLIFERATION PHASE

RECONSTRUCTION PHASE

MACROPHAGES

FIBROBLASTS COLLAGEN

MACROPHAGES

# FIG. 6

WOUND CREATION POSITION

AS THE INFILTRATION OF THE INFLAMMATORY CELLS WAS NOT OBSERVED
AND GRANULATION TISSUE (CAPILLARY VESSELS AND COLLAGEN FIBER FORMATION)
WAS OBSERVED, IT WAS DETERMINED TO BE AT A PROLIFERATION PHASE.

EP 3 552 609 B1

# FIG. 7

SIMONKOLLEITE

pH AFTER DISSOLUTION TEST vs pH AT SYNTHESIS

# FIG. 8

SIMONKOLLEITE

AMOUNT OF DISSOLVED $Zn^{2+}$ IONS ($\mu g/m^{-2}$) vs pH AT SYNTHESIS

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012531421 A

**Non-patent literature cited in the description**

- **MAVROMICHALIS I. et al.** *Can. J. Anim. Sci.,* 2001, vol. 81, 387-391

- **COUSY S. et al.** *Nanocon,* 2013